(19) Europäisches Patentamt
European Patent Office
Office européen des brevets

(11) Veröffentlichungsnummer: **0 289 910**
**A2**

(12) # EUROPÄISCHE PATENTANMELDUNG

(21) Anmeldenummer: **88106629.4**

(22) Anmeldetag: **26.04.88**

(51) Int. Cl.⁴: **C07D 209/48 , A01N 43/38 , C07D 405/12**

Patentansprüche für folgenden Vertragsstaat: ES.

(30) Priorität: **30.04.87 DE 3714373**

(43) Veröffentlichungstag der Anmeldung: **09.11.88 Patentblatt 88/45**

(84) Benannte Vertragsstaaten: **AT BE CH DE ES FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Schlegel, Günter, Dr.**
**Feldbergstrasse 18**
**D-6237 Liederbach(DE)**
Erfinder: **Mildenberger, Hilmar, Dr.**
**Fasanenstrasse 24**
**D-6233 Kelkheim (Taunus)(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**

(54) **Substituierte Tetrahydrophthalimide, Verfahren zu ihrer Herstellung und ihre Verwendung als Herbizide.**

(57) Verbindungen der Formel I,

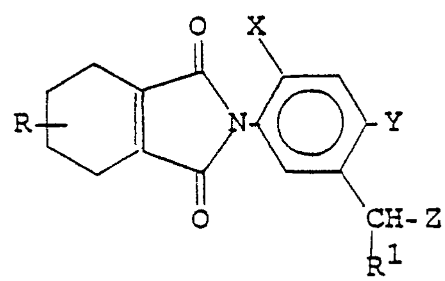

(I)

worin
X = Wasserstoff oder Halogen, Y = Halogen

**EP 0 289 910 A2**

Xerox Copy Centre

Z =

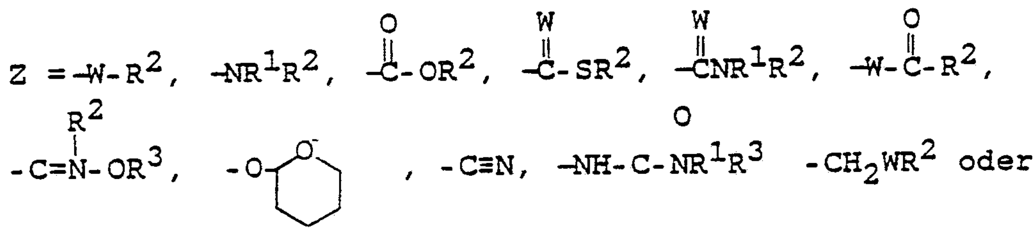

oder

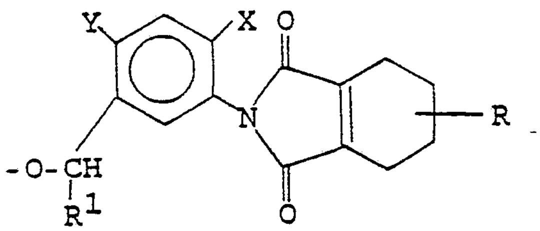

R = H oder Alkyl, R' = Wasserstoff, Hydroxy, Alkyl, Alkoxy, Alkylthio, (subst.) Amino, Cyano, oder R' und Z zusammen einen Rest $-W-CH_2-CH_2-W-$bedeuten,besitzen vorteilhafte herbizide Wirkungen.

## Substituierte Tetrahydrophthalimide, Verfahren zu ihrer Herstellung und ihre Verwendung als Herbizide

Es ist bereits bekannt, daß Tetrahydrophthalimide, die bestimmte Benzylgruppen mit Phosphorsäureesterresten enthalten, herbizide Eigenschaften besitzen, s. JP-A 1103-887. Diese Verbindungen weisen jedoch Nachteile bei der Anwendung auf, wie z.B. eine nicht ausreichende Selektivität oder eine zu geringe Wirkungsbreite.

Es wurden nun neue Tetrahydrophthalaimide mit anderen Substituenten an einem benzylischen Kohlenstoffatom gefunden, die überraschenderweise eine vorteilhaft selektive Herbizidwirkung besitzen.

Gegenstand der vorliegenden Erfindung sind daher die Verbindungen der Formel (I) oder deren Salze

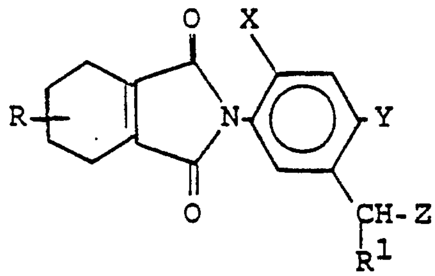

(I),

worin
X = Wasserstoff oder Halogen,
Y = Halogen

Z = 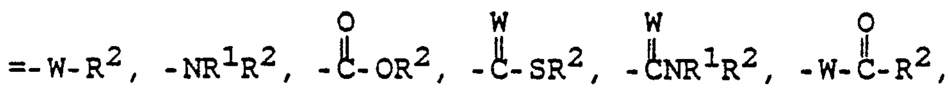

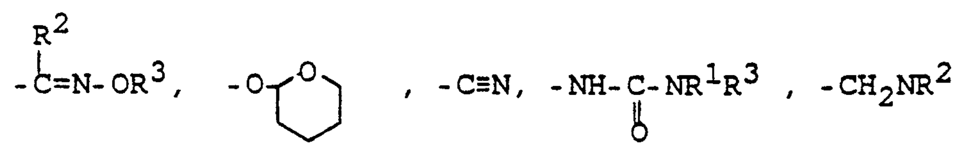 oder

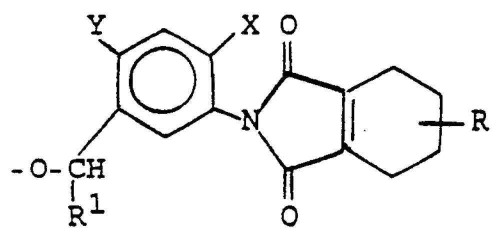

R = H oder ($C_1$-$C_4$)-Alkyl
$R^1$ = Wasserstoff, Hydroxy, ($C_1$-$C_4$)-Alkyl, ($C_1$-$C_4$)-Alkoxy, ($C_1$-$C_4$)-Alkylthio, Amino, ($C_1$-$C_4$)-Alkylamino, ($C_1$-$C_4$)-Dialkylamino oder Cyano,
oder $R^1$ und Z zusammen einen Rest -W-$CH_2$-$CH_2$-W-
$R^2$ = Wasserstoff, ($C_2$-$C_4$)-Alkenyl, ($C_2$-$C_4$)-Alkinyl, wobei der Alkyl-, Alkenyl-oder Alkinyl-Rest jeweils durch ($C_3$-$C_7$)-Cycloalkyl, Halogen, ($C_1$-$C_4$)-Alkoxy, ($C_1$-$C_4$)-Alkylthio, Di-($C_1$-$C_4$-alkyl)amino, Cyano, Nitro, Phenyl, Tri-($C_1$-$C_4$-alkyl)silyl, Benzyloxy oder durch einen vier-bis siebengliedrigen gesättigten oder ungesättigten ein bis drei Stickstoff-, Sauerstoff-oder Schwefelatome enthaltenden Heterocyclus substituiert sein kann, ($C_3$-$C_7$)-Cycloalkyl, unsubstituiertes oder durch Halogen, Cyano, ($C_1$-$C_4$)-Alkyl, ($C_1$-$C_4$)-Alkoxy oder Phenoxy, das durch $CF_3$, Halogen oder ($C_1$-$C_4$)-Alkyl substituiert sein kann, substituiertes Phenyl, ferner einen Rest der Formeln

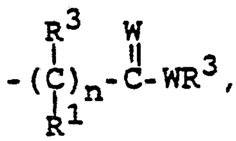, 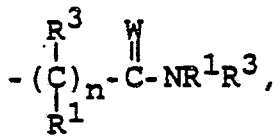, 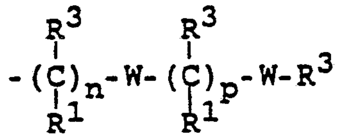,

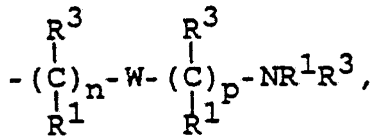, 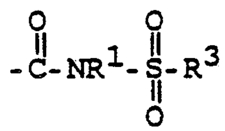, 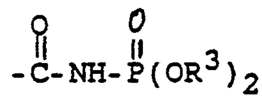,

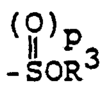 oder 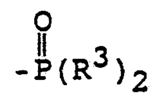,

$R^3$ = Wasserstoff, ($C_1$-$C_4$)-Alkyl, ($C_3$-$C_7$)-Cycloalkyl, ($C_2$-$C_4$)-Alkenyl, ($C_2$-$C_4$)-Alkinyl oder Phenyl, das durch Halogen, Cyano, ($C_1$-$C_4$)-Alkyl oder ($C_1$-$C_4$)-Alkoxy substituiert sein kann,
n = 0, 1 oder 2
p = 1 oder 2 und
W = Sauerstoff oder Schwefel
bedeuten.

Bevorzugt hiervon sind Verbindungen der Formel I, worin
X = H, F oder Cl
Y = F, Cl oder Br

Z = $WR^2$, 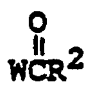 oder 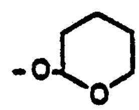, insbesondere $WR^2$,

$R^1$ = H, ($C_1$-$C_4$)-Alkyl oder ($C_1$-$C_4$)-Alkoxy
$R^2$ = H, ($C_1$-$C_4$)-Alkyl, ($C_2$-$C_4$)-Alkenyl, ($C_2$-$C_4$)-Alkinyl, die durch ($C_1$-$C_4$)-Alkoxy, ($C_1$-$C_4$)-Alkylthio oder Halogen substituiert sein können oder einen Rest der Formeln

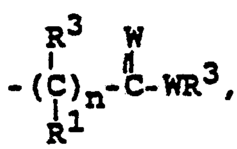, 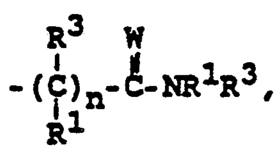, 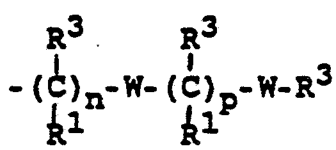,

$R^3$ = H, ($C_1$-$C_4$)-Alkyl oder Phenyl
n = 0, 1 oder 2
p = 1 oder 2 und
W = O oder S bedeuten.

Alkyl, Alkenyl, Alkinyl oder Alkoxy kann in obengenannten Definitionen geradkettig oder verzweigt sein. Halogen bedeutet bevorzugt Fluor, Chlor oder Brom.

Im Falle $R^2$ oder $R^3$ = Wasserstoff können die Verbindungen als Salze vorliegen. Landwirtschaftlich einsetzbare Salze sind beispielsweise Na-, K-, Mg-, Ca-, Zn-Salze oder Ammonium-oder organische mono- bis tetrasubstituierte Ammonium-, Sulfonium-, Sulfoxonium-oder Phosphonium-Salze, wobei als Substituierten beispielsweise Alkyl-, Hydroxyalkyl-, Cycloalkyl-, Phenyl-Reste etc. fungieren können.

Die Verbindungen der Formel (I) besitzen am benzylischen -CHR¹Z für $R^1 \neq H \neq Z$ ein optisch aktives Zentrum. Sie können daher als Stereoisomerengemische oder in Form der reinen Stereoisomeren vorliegen oder mit weiteren optisch aktiven Substituenten Diastereomere bilden. Die Erfindung erfaßt alle diese Isomeren oder deren Gemische.

Gegenstand der vorliegenden Erfindung sind auch Verfahren zur Herstellung der Verbindungen der Formel (I) und deren Salze, dadurch gekennzeichnet, daß man

a) eine Verbindung der Formel (II) mit einer Verbindung der Formel (III)

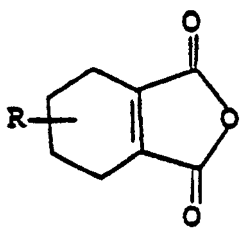

II

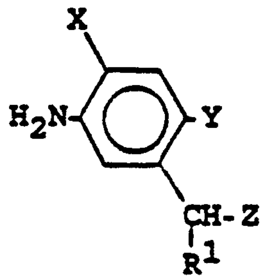

III

oder

b) ein Isocyanat der Formel (IV) mit einer Verbindung der Formel (II)

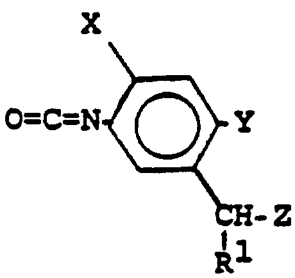

IV

oder

c) eine Verbindung der Formel (V),

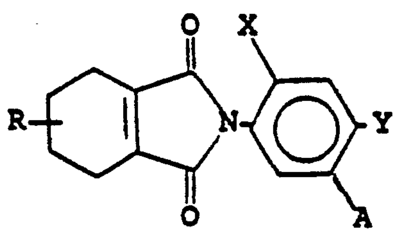

V

worin A = einen Rest der Formeln-CHO, $-CHR^1-Hal$ (Hal = Cl oder Br), $-CHR^1-OH$ oder $-CHR^1-COOR^3$ bedeutet, in an sich bekannter Weise zu den Verbindungen der Formel I derivatisiert und die unter a) bis c) erhaltenen Verbindungen gegebenenfalls in ihre Salze überführt.

Im Falle der Verfahrensvariante a) arbeitet man zweckmäßigerweise in einem inerten Lösungsmittel beispielsweise einem aromatischen Lösungsmittel wie Toluol, Chlorbenzol oder Dichlorbenzol oder in Eisessig. Es ist jedoch auch möglich, auf das Lösungsmittel zu verzichten. Gegebenenfalls kann ein Katalysator wie p-Toloulsulfonsäure zugesetzt werden. Die Reaktionstemperaturen können zwischen 10°C und 160°C variieren. Bei diesen Temperaturen erfolgt auch die Umsetzung für die Variante b). In letzterem Falle werden als inerte Lösungsmittel beispielsweise aromatische Lösungsmittel wie Toluol, Xylol verwendet. Man kann auch ohne Lösungsmittel arbeiten.

Als Derivatisierungsreaktionen im Falle c) kommen beispielsweise Veresterungen, Alkylierungen, Acylierungen oder nucleophile Substitutionen infrage, die als Standardreaktionen dem Fachmann geläufig sind. Ebenso erfolgt die Salzbildung nach üblichen Methoden.

Die Ausgangsverbindungen der Formel II oder III sind bekannt oder nach üblichen Methoden zugänglich. Die Verbindungen der Formel V werden analog den Verfahrensvarianten a) oder b) aus den Verbindungen der Formel II hergestellt.

Die erfindungsgemäßen Verbindungen der Formel I weisen eine ausgezeichnete herbizide Wirksamkeit gegen ein breites Spektrum wirtschaftlich wichtiger mono-und dikotyler Schadpflanzen auf. Auch schwer bekämpfbare perennierende Unkräuter, die aus Rhizomen, Wurzelstöckchen oder anderen Dauerorganen

austreiben, werden durch die Wirkstoffe gut erfaßt. Beispiels für solche Schadpflanzen sind verschiedene Arten von Avena, Lolium, Alopecurus, Phalaris, Echinochloa, Digitaria, Setaria, Cyperus, Agropyron, Cynodon, Imperata, Sorghum, Galium, Viola, Veronica, Lamium, Stellaria, Amaranthus, Sinapis, Ipomoea, Matricaria, Abutilon. Sida, Convolvulus, Cirsium, Rumex, Artemisia, Sagittaria, Alisma, Eleocharis und Scirpus. Dabei ist es gleichgültig, ob die Substanzen im Vorsaat-, Vorauflauf-oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf dies Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono-und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerliechterung wie z.B. durch Auslösen von Desikkation, Abszission und Wuchsstauchung eingesetzt werden. Desweiteren eignen sie sich auch zur generellen Steuerung und Hemmung von unerwünschtem begatativen Wachstum, ohne dabei die Pflanzen abzutöten. Eine Hemmung des vegatativen Wachstums spielt bei vielen mono-und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs-oder Inertstoff noch Netzmittel, z.B. polyoxethylierte Alkylphenole, polyoxethylierte Fettalkohole, Alkyl-oder Alkylphenylsulfonate und Dispergiermittel, z.B. ligninsulfonsaures Natrium, 2.2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial, Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% and Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel,

Füllstoffe usw. verwendet werden.

Daneben enthalten die genannten Wirkstofformulierungen gegebenenfalls die jeweils üblichen Haft-, Netz-. Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitenungen sowie versprühbare Lösungen werden vor der Anwendung üblicherweise nicht mehr mit weiteren inerten Stoffwen verdünnt.

Mit den äußeren Bedingungen wie Temperatur, Feuchtigkeit u.a. variiert die erforderliche Aufwandmenge. Sie kann innerhalb wieter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngsmitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz aus Inerstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (Triton x 207), 3 Gewichtsteilen Isotridecanolpolyglykolether (8 AeO) und 71 Gewichtsteilen paraffinischem Mineralöl (Siedebereich z.B. ca. 255 bis über 377°C) mitscht und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

**Herstellungsbeispiele**

**A. Vorprodukte**

**Verbindung 1**

N-(40Chlor-3-hydroxymethyl)-phenyl-4,5,6,7-tetrahydroisoindolin-1,3-dion

Eine Lösung von 26,0 g (0,17 mol) 4-Chlor-3-hydroxymethylanilin und 30,4 g (0,20 mol) 3,4,5,6-Tetrahydrophthalsäureanhydrid in 100 ml Eisessig wurde 5 Stunden unter Rückfluß erhitzt. Nach Zugabe von 100 ml Wasser hielt man das Reaktionsgemisch 2 Stunden bei 60°C und saugte nach Abkühlen den Feststoff ab. Man erhielt 22,5 g (46 % d.Th.) N-(4-Chlor-3-hydroxymethyl)-phenyl-4,5,6,7-tetrahydroisoindolin-1,3,-dion vom Schmp. 125 - 127°C.

**Verbindung 2**

N-(2,4-Dichlor-5-hydroxymethyl)-phenyl-4,5,6,7-tetrahydroisoindolin-1,3-dion

Man erhitzte eine Lösung von 50 g (0,26 mol) 2,4-Dichlor-5-hydroxymethyl-anilin, 49,5 g (0,33 mol) 3,4,5,6-Tetrahydrophthalsäureanhydrid und 1,0 g p-Toluolsulfonsäure in 350 ml o-Dichlorbenzol 2 Stunden unter Rückfluß. Nach Einengen im Vakuum wurde der Rückstand aus Ethanol/Isopropylether/Hexan (3/3/6) umkristallisiert. Ausbeute: 62,6 g (74 % d.Th.) vom Schmp. 168-170°C.

**Verbindung 3**

N-(4-Chlor-2-fluor-5-hydroxymethyl)-phenyl-4,5,6,7-tetrahydroisoindolin-1,3-dion

35 g (0,20 mol) 4-Chlor-2-fluor-5-hydroxymethyl-anilin und 35 g (0,23 mol) 3,4,5,6-Tetrahydrophthalsäureanhydrid wurden in 400 ml Toluol gelöst. Nach Zugabe von 0,5 g p-Toluolsulfonsäure wurde am Wasserabscheider 9 Stunden unter Rückfluß erhitzt. Man engte im Vakuum ein und kristallisierte das Produkt durch Rühren des Rückstandes in Isopropylether. Es wurden 37,6 g (61 % d.Th.) N-(4-Chlor-2-Fluor-5-hydroxymethyl)-phenyl-4,5,6,7-tetrahydroisoindolin-1,3-dion vom Schmp. 161-163°C gewonnen.

**B. Endprodukte**

**Beispiel 1**

N-Ethyl-[2-Chlor-5-(4,5,6,7-tetrahydroisoindolin-1,3-dion-2-yl)]-benzylcarbamat

4,5 g (0,015 mol) N-(4-Chlor-3-hydroxymethyl)-phenyl-4,5,6,7-tetrahydroisoindolin-1,3-dion (s. Verbindung 1) wurden in 25 ml Acetonitril gelöst und bei Raumtemperatur mit 2.4 ml (0,030 mol) Ethylisocyanat versetzt. Nach Zugabe von 1 Tropfen Triethylamin erhitzte man 8 Stunden auf 60°C, engte ein und rührte den Rückstand in Isopropylether aus. Man erhielt 3,5 g (65 % d.Th.) N-Ethyl-[2-Chlor-5-(4,5,6,7-tetrahydroisoindolin-1,3-dion-2-yl)]-benzylcarbamat vom Schmp. 67-69°C.

**Beispiel 2**

N-[4-Chlor-2-fluor-5-(methoxy-methoxy)-methyl]-phenyl-4,5,6,7-tetrahydroisoindolin-1,3-dion

3,1 g (0.01 mol) N-(4-Chlor-2-fluor-5-hydroxymethyl)-phenyl-4,5,6,7-tetrahydroisoindolin-1,3-dion (s. Verbindung 3) wurden in 30 ml Dimethoxymethan gelöst und portionsweise mit 3 g Phosphorpentoxid und 0,1 g Lithiumbromid versetzt. Nach fünfstündigem Rühren bei Raumtemperatur gab man die Reaktionsmischung in 60 ml gesättigte Natriumhydrogencarbonatlösung und extrahierte 2 x mit je 50 ml Diethylether. Die vereinigten organischen Extrakte wurden über Natriumsulfat getrocknet und im Wasserstrahlvakuum eingeengt. Es blieben 2,9 g (82 %) N-[4-Chlor-2-fluor-5-(methoxy-methoxy)-methyl]-phenyl-4,5,6,7-tetrahydroisoindolin-1,3-dion als Harz zurück.

Auf analoge Weise werden die folgenden Verbindungen der Formel I hergestellt:

**Tabelle 1**

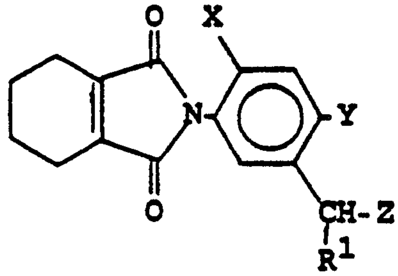

| Bsp. Nr. | X | Y | $R^1$ | Z | Schmp. [°C] |
|---|---|---|---|---|---|
| 3 | Cl | Cl | $CH_3$ | OH | |
| 4 | Cl | Cl | H | 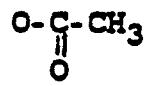 | 63/65 |
| 5 | Cl | Cl | H | 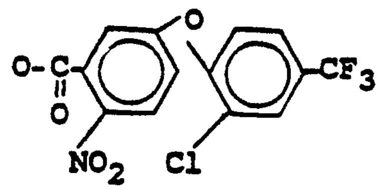 | 65/66 |
| 6 | Cl | Cl | H | 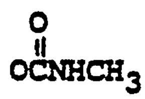 | 141/43 |

Fortsetzung Tabelle 1

| Bsp. Nr. | X | Y | $R^1$ | Z | Schmp. [°C] |
|---|---|---|---|---|---|
| 7 | Cl | Cl | H | $O\overset{\overset{O}{\|}}{C}CH_2Cl$ | Öl |
| 8 | Cl | Cl | H | $O\overset{\overset{O}{\|}}{C}O\text{-}C_6H_5$ | Öl |
| 9 | Cl | Cl | H | $O\overset{\overset{O}{\|}}{C}C_2H_5$ | Öl |
| 10 | Cl | Cl | H | $OSO_2CH_3$ | 122/24 |
| 11 | Cl | Cl | OH | $O\text{-}\overset{\overset{O}{\|}}{C}CH_3$ | |
| 12 | Cl | Cl | H | $O\text{-}\overset{\overset{O}{\|}}{C}CF_3$ | Öl |
| 13 | Cl | Cl | H | $O\text{-}\overset{\overset{O}{\|}}{C}CCl_3$ | 110/12 |
| 14 | Cl | Cl | H | $OCH_2Cl$ | Öl |
| 15 | Cl | Cl | H | $O\overset{\overset{O}{\|}}{C}CH_2OCH_3$ | Öl |
| 16 | Cl | Cl | $OCH_3$ | $OCH_3$ | Öl |
| 17 | Cl | Cl | H | $OCH_2OCH_3$ | 61/63 |
| 18 | Cl | Cl | OH | $\overset{\overset{O}{\|}}{P}(CH_3)_2$ | 219/21 |
| 19 | Cl | Cl | H | 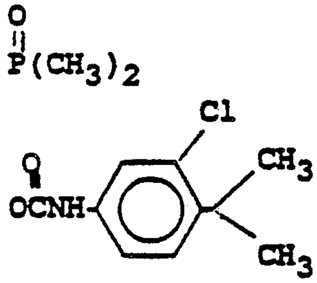 | 180/82 |

Fortsetzung Tabelle 1

| Bsp. Nr. | X | Y | $R^1$ | Z | Schmp. [°C] |
|---|---|---|---|---|---|
| 20 | Cl | Cl | H | $O\overset{O}{\overset{\|}{C}}NH-CH(CH_3)_2$ | 152/53 |
| 21 | Cl | Cl | H | $O\overset{O}{\overset{\|}{C}}NH-C_2H_5$ | 158/59 |
| 22 | H | Cl | $C_2H_5$ | OH | |
| 23 | Cl | Cl | H | 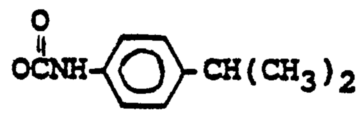 | 164/66 |
| 24 | Cl | Cl | H | 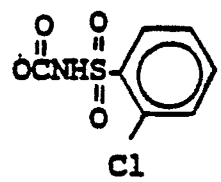 | 92/94 |
| 25 | H | Cl | H | $O\overset{O}{\overset{\|}{C}}C_2H_5$ | Öl |
| 26 | Cl | Cl | H | $O\overset{O}{\overset{\|}{C}}$-cyclo-$C_3H_5$ | 92/94 |
| 27 | H | Cl | H | $O\overset{O}{\overset{\|}{C}}NH-CH_3$ | 79/81 |
| 28 | F | Cl | $CH_3$ | OH | |
| 29 | H | Cl | $CH_3$ | $O\overset{O}{\overset{\|}{C}}NHC_2H_5$ | |
| 30 | Cl | Cl | H | $O\overset{O}{\overset{\|}{C}}NHCH_2CH_2CH_3$ | 101/03 |
| 31 | Cl | Cl | H | $O\overset{O}{\overset{\|}{C}}-C(CH_3)_3$ | Öl |

Fortsetzung Tabelle 1

| Bsp. Nr. | X | Y | $R^1$ | Z | Schmp. [°C] |
|---|---|---|---|---|---|
| 32 | Cl | Cl | H | $O\overset{O}{\overset{\|}{C}}$-Cyclo-$C_4H_7$ | Öl |
| 33 | Cl | Cl | H | $O\overset{O}{\overset{\|}{C}}$-Cyclo-$C_6H_{11}$ | Öl |
| 34 | Cl | Cl | H | $O\overset{O}{\overset{\|}{C}}NH\overset{O}{\overset{\|}{P}}(OCH_3)_2$ | 134/36 |
| 35 | F | Cl | H | $O\overset{O}{\overset{\|}{C}}NHC_2H_5$ | 93/100 |
| 36 | F | Cl | H | $OCH_2SCH_3$ | |
| 37 | H | Cl | H | $OCH_2OCH_3$ | 52/53 |
| 38 | H | Cl | H | $O\overset{O}{\overset{\|}{C}}NH\overset{O}{\overset{\|}{P}}(OC_2H_5)_2$ | 66/68 |
| 39 | H | Cl | H | $O\overset{O}{\overset{\|}{C}}$-Cyclo-$C_3H_5$ | Öl |
| 40 | H | Cl | H | $O\overset{O}{\overset{\|}{C}}$-Cyclo-$C_6H_{11}$ | 57/59 |
| 41 | H | Cl | H | $O\overset{O}{\overset{\|}{C}}-C(CH_3)_3$ | 60/62 |
| 42 | F | Cl | H | $O\overset{O}{\overset{\|}{C}}-C_2H_5$ | Öl |
| 43 | H | Cl | H | $O\overset{O}{\overset{\|}{C}}NH\overset{O}{\overset{\|}{P}}(OCH_3)_2$ | 183/85 |

Fortsetzung Tabelle 1

| Bsp. Nr. | X | Y | $R^1$ | Z | Schmp. [°C] |
|---|---|---|---|---|---|
| 44 | H | Cl | H | 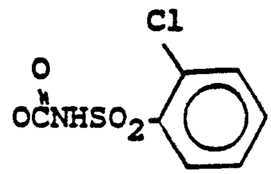 | 163/65 |
| 45 | Cl | Cl | H | COOH | 216/18 |
| 46 | Cl | Cl | H | $COOC_2H_5$ | 80/82 |
| 47 | Cl | Cl | H | CONH-n-$C_4H_9$ | 216/19 |
| 48 | Cl | Cl | H | $COOCH_2CF_2CHF_2$ | 81/83 |
| 49 | Cl | Cl | H | $COOCH_3$ | 84/85 |
| 50 | Cl | Cl | H | COO-$C_6H_5$ | 176/80 |
| 51 | Cl | Cl | H | 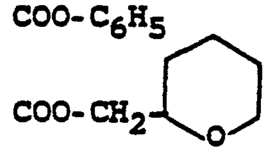 | Wachs |
| 52 | Cl | Cl | H | 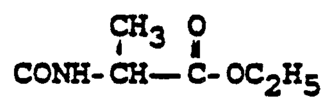 | 181/83 |
| 53 | Cl | Cl | H | CONH-$CH_2$-$C_6H_5$ | 226/28 |
| 54 | Cl | Cl | H | $CON(CH_3)_2$ | 141/44 |
| 55 | Cl | Cl | H | $COOCH_2C{\equiv}CH$ | 110/12 |
| 56 | Cl | Cl | H | $CONHCH_3$ | 225/26 |
| 57 | Cl | Cl | H | 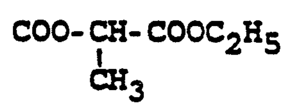 | Wachs |
| 58 | Cl | Cl | H | COONa | 147/50 |

Fortsetzung Tabelle 1

| Bsp. Nr. | X | Y | $R^1$ | Z | Schmp. [°C] |
|---|---|---|---|---|---|
| 59 | Cl | Cl | H | 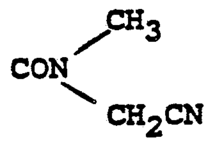 | 193/96 |
| 60 | Cl | Cl | H | $COOCH_2CH=CH_2$ | Wachs |
| 61 | Cl | Cl | H | 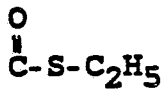 | Wachs |
| 62 | Cl | Cl | H | 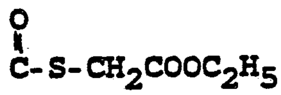 | Wachs |
| 63 | Cl | Cl | H | 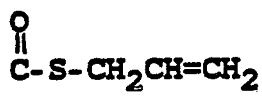 | Wachs |
| 64 | Cl | Cl | H | 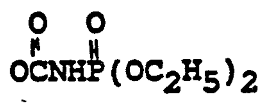 | 107/09 |
| 65 | F | Cl | H | 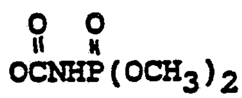 | 126/28 |
| 66 | H | Cl | H | 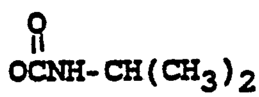 | 92/94 |
| 67 | F | Cl | H | COOH | 182/84 |
| 68 | F | Cl | H | 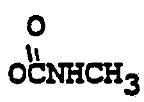 | 65/68 |
| 69 | F | Cl | H | COONa | 82/84 |
| 70 | F | Cl | H | $COOCH_2CH_2OCH_3$ | 60/62 |

Fortsetzung Tabelle 1

| Bsp. Nr. | X | Y | $R^1$ | Z | Schmp. [°C] |
|---|---|---|---|---|---|
| 71 | F | Cl | H | $CONHCH_2CH_2OCH_3$ | 162/64 |
| 72 | F | Cl | H | $COOCH_3$ | 101/03 |
| 73 | Cl | Cl | H | $O-\overset{S}{\overset{\|}{C}}NH-(CH_2)_3CH_3$ | 163/65 |
| 74 | F | Cl | H | $O-CH(CH_3)-OCH_3$ | 109/111 |
| 75 | Cl | Cl | H | $OCH_2OC_2H_5$ | Wachs |
| 76 | Cl | Cl | H | $O-CH(CH_3)-OCH_3$ | Wachs |
| 77 | Cl | Cl | H | $\overset{O}{\overset{\|}{C}}-ON=C(CH_3)-COOC_2H_5$ | Wachs |
| 78 | Cl | Cl | H | $OCH(CH_3)-OC_2H_5$ | Wachs |
| 79 | Cl | Cl | H | $OCH_3$ | 77-79 |
| 80 | Cl | Cl | H | 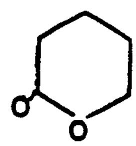 | Wachs |
| 81 | F | Cl | H | $OCH_3$ | 79-81 |
| 82 | F | Cl | H | $OCH_2OC_2H_5$ | Wachs |
| 83 | F | Cl | $OCH_3$ | $OCH_3$ | Wachs |
| 84 | F | Cl | H | $OCH(CH_3)OC_2H_5$ | Wachs |
| 85 | Cl | Cl | H | $OC_2H_5$ | Wachs |

Fortsetzung Tabelle 1

| Bsp. Nr. | X | Y | $R^1$ | Z | Schmp. [°C] |
|---|---|---|---|---|---|
| 86 | F | Cl | H | $O\overset{\overset{O}{\|}}{C}-C_6H_5$ | 145/47 |
| 87 | F | Cl | H | $O\overset{\overset{O}{\|}}{C}-CH(CH_3)_2$ | Wachs |
| 88 | F | Cl | H | $O\overset{\overset{O}{\|}}{C}-CH_3$ | Wachs |
| 89 | Cl | Cl | $-O-CH_2CH_2O-$ | | Wachs |
| 90 | F | Cl | H | $O\overset{\overset{O}{\|}}{C}NH(CH_2)_3CH_3$ | Wachs |
| 91 | H | Br | H | $OCH_3$ | |
| 92 | F | Br | H | $OCH_3$ | |
| 93 | F | Br | H | $OCH_2OCH_3$ | |
| 94 | H | Br | $CH_3$ | $OCH(CH_3)OCH_3$ | |
| 95 | H | Br | $OCH_3$ | $OCH_3$ | |
| 96 | F | F | H | $OCH_2OC_2H_5$ | |
| 97 | F | Cl | H | $SCH_3$ | |
| 98 | F | Cl | $CH_3$ | $SCH_2COOC_2H_5$ | |
| 99 | Cl | Cl | H | $N(CH_3)_2$ | |
| 100 | F | Cl | H | $NHCH_2CONH_2$ | |

Fortsetzung Tabelle 1

| Bsp. Nr. | X | Y | $R^1$ | Z | Schmp. [°C] |
|---|---|---|---|---|---|
| 101 | H | Cl | $CH_3$ | $NHCH(CH_3)CONHCH_3$ | |
| 102 | H | Br | $SCH_3$ | $SCH_3$ | |
| 103 | Cl | Br | H | $NHCH_3$ | |
| 104 | H | Cl | H | $COOCH(CH_3)-OC_2H_5$ | |
| 105 | H | Cl | $CH_3$ | $COOCH_2OCH_2-C_6H_5$ | |
| 106 | H | Cl | H | $CONHCH_2-C_6H_5$ | |
| 107 | H | Cl | $CH_3$ | $CONH(CH_2)_2OCH_3$ | |
| 108 | Cl | Cl | CN | CN | |
| 109 | Cl | Cl | $CH_3NH$ | $CH_3NH$ | |
| 110 | F | Cl | $C_2H_5NH$ | $C_2H_5NH$ | |
| 111 | F | Cl | $CH_3NH$ | $COOC_2H_5$ | |
| 112 | F | Cl | $CH_3NH$ | $COOCH_2CH_2OCH_3$ | |
| 113 | F | Cl | $C_2H_5NH$ | $CONH-CH_2CH_2OC_2H_5$ | |
| 114 | F | Cl | $C_2H_5NH$ | $CSNHCH_3$ | |
| 115 | F | Cl | $C_2H_5NH$ | $NH\underset{O}{\underset{\|}{C}}NHCH_3$ | |
| 116 | F | Cl | $CH_3$ | $NH\underset{O}{\underset{\|}{C}}NHCH_3$ | |

Fortsetzung Tabelle 1

| Bsp. Nr. | X | Y | $R^1$ | Z | Schmp. [°C] |
|---|---|---|---|---|---|
| 117 | F | Cl | $CH_3$ | $NHCNH-OCH_3$<br>‖<br>O | |
| 118 | F | Cl | $CH_3$ | $NHCNH-(CH_2)_2CH_3$<br>‖<br>S | |
| 119 | F | Cl | $CH_3$ | $NHCNHCH(CH_3)_2$<br>‖<br>O | |
| 120 | F | Cl | CN | CN | |
| 121 | F | Cl | OH | $CH_3$ | |
| 122 | F | Cl | OH | $CH_2COOC_2H_5$ | |
| 123 | F | Cl | OH | $SCH_3$ | |
| 124 | F | Cl | OH | $CH_2CH_2OCH_3$ | |
| 125 | F | Cl | OH | $CH_2COOCH_2-C_6H_5$ | |
| 126 | H | Br | OH | $OC_2H_5$ | |
| 127 | H | Br | OH | $SCCl_3$ | |
| 128 | H | Br | OH | OH | |
| 129 | F | F | H | $OCH_3$ | |
| 130 | F | F | H | $OCH_2OCH_3$ | |
| 131 | F | F | H | $OCH(CH_3)OCH_3$ | |

Fortsetzung Tabelle 1

| Bsp. Nr. | X | Y | $R^1$ | Z | Schmp. [°C] |
|---|---|---|---|---|---|
| 132 | F | F | H | $OCH_2CH_2SCH_3$ | |
| 133 | F | F | H | $OCH_2CH_2N(CH_3)_2$ | |
| 134 | F | F | H | $COOCH_2CH_2OCH_3$ | |
| 135 | F | F | H | $COOCH_2CH_2OSi(CH_3)_3$ | |
| 136 | F | F | H | $CONHCH_3$ | |
| 137 | F | F | H | $CONH(CH_2)_2OCH_3$ | |
| 138 | F | F | $CH_3$ | $CONH(CH_2)_2C(OCH_3)_2$ | |
| 139 | F | F | $CH_3$ | $CSNHCH_2CH(CH_3)_2$ | |
| 140 | F | F | $CH_3$ | $OCH_2CH_2$- 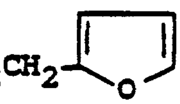 | |
| 141 | F | F | $CH_3$ | $OCH_2$- 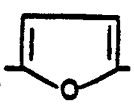 -$CH_3$ | |
| 142 | H | CL | H | $CONHCH_2$- 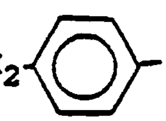 - Cl | |
| 143 | H | Cl | $CH_3$ | $CONHCH_2$- 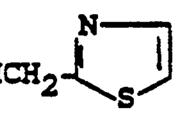 | |
| 144 | H | Cl | $C_2H_5$ | CONH-$CH_2$- 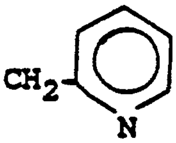 | |
| 145 | H | F | $C_2H_5$ | $COOCH_2$- 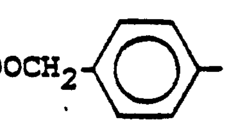 - $OCH_3$ | |

Fortsetzung Tabelle 1

| Bsp. Nr. | X | Y | $R^1$ | Z | Schmp. [°C] |
|---|---|---|---|---|---|
| 146 | H | F | $C_2H_5$ | $OCH_2CH_2$- 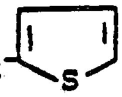 | |
| 147 | Cl | Cl | H | S-$CH_2$- 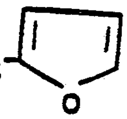 | |
| 148 | Cl | Cl | H | O-$CH_2$- 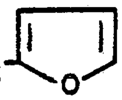 | |
| 149 | Cl | Cl | H | O-$CH_2$- 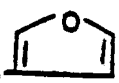 | |
| 150 | F | Cl | H | $OCH_2CH_2CN$ | |
| 151 | F | Cl | H | $OCH_2CH_2CH_2-NO_2$ | |
| 152 | F | Cl | $CH_3$ | $OCH_2CH_2Cl$ | |
| 153 | F | Cl | $CH_3$ | $O(CH_2)_3CH_2Br$ | |
| 154 | F | Cl | $CH_3$ | $OCH_2CH_2\overset{S}{\overset{\|}{C}}-S-CH_3$ | |
| 155 | H | Cl | H | $OCH(CH_3)-CH_2Cl$ | |
| 156 | H | Cl | H | $OCH(CH_3)-COOC_2H_5$ | |
| 157 | H | Cl | H | $O\overset{O}{\overset{\|}{C}}NHCH_2CN$ | |
| 158 | H | Cl | H | $O\overset{O}{\overset{\|}{C}}NHCH(CH_3)CN$ | |

Fortsetzung Tabelle 1

| Bsp. Nr. | X | Y | $R^1$ | Z | Schmp. [°C] |
|---|---|---|---|---|---|
| 159 | H | Cl | H | $S-\overset{O}{\overset{\|}{C}}NH(CH_2)_4CH_3$ | |
| 160 | H | Cl | H | $OCH_2OCH_2$- 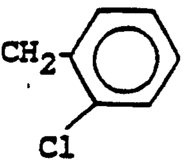 | |
| 161 | H | Cl | $SCH_3$ | $COOCH_3$ | |
| 162 | F | Cl | $SCH_3$ | $SCH_3$ | |
| 163 | F | Cl | H | $COOCH_2-S-CH_2COOC_2H_5$ | |
| 164 | F | Cl | $-S-CH_2-CH_2-S-$ | | |
| 165 | H | Cl | H | 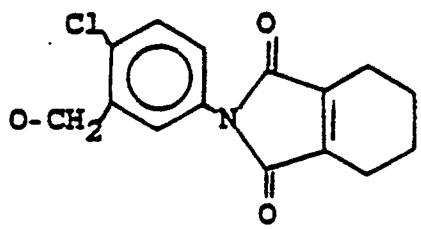 | |
| 166 | F | Cl | H | 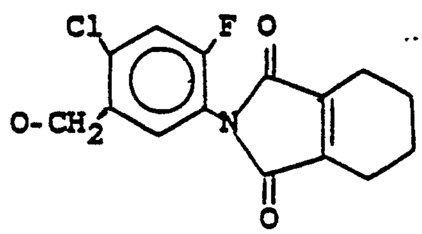 | |
| 167 | Cl | Cl | H | 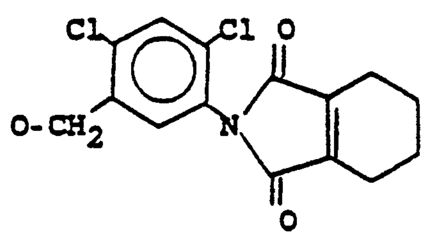 | |

Fortsetzung Tabelle 1

| Bsp. Nr. | X | Y | $R^1$ | Z | Schmp. [°C] |
|---|---|---|---|---|---|
| 168 | H | Br | $CH_3$ | 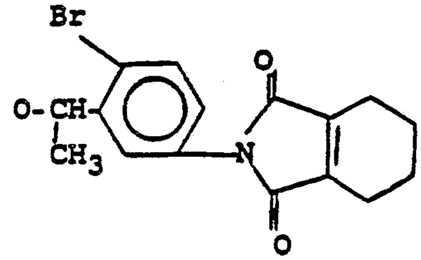 | |
| 169 | H | Cl | H | 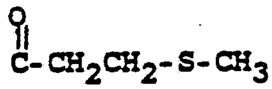 | |
| 170 | H | Cl | H | 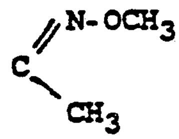 | |
| 171 | F | Cl | H | 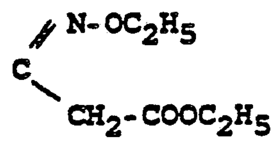 | |
| 172 | F | Cl | $CH_3$ | $OCH_2CH{=}CH_2$ | |
| 173 | F | Cl | $CH_3$ | $OCH_2C{\equiv}CH$ | |
| 174 | F | Cl | H | $OCH_2CHCl-CH_2Cl$ | |
| 175 | F | Cl | $NHCH_3$ | $NHCH_2CH{=}CH_2$ | |
| 176 | F | Cl | $CH_3$ | 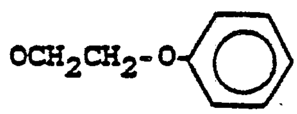 | |
| 177 | F | Cl | H | 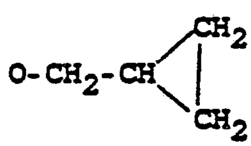 | |
| 178 | F | Br | $SCH_3$ | 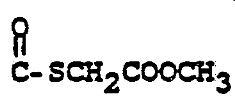 | |
| 179 | F | Br | $S(CH_2)_3CH_3$ | $S(CH_2)_3CH_3$ | |
| 180 | F | J | H | $OCH_3$ | |

Fortsetzung Tabelle 1

| Bsp. Nr. | X | Y | $R^1$ | Z | Schmp. [°C] |
|---|---|---|---|---|---|
| 181 | H | Br | H | $O\overset{O}{\overset{\|}{C}}NHC_2H_5$ | 88-90 |
| 182 | H | Cl | H | $OCH_3$ | 88-90 |
| 183 | F | Cl | H | $O\overset{O}{\overset{\|}{C}}NHCH(CH_3)_2$ | 116-118 |
| 184 | H | Cl | $OCH_3$ | $OCH_3$ | 87-89 |
| 185 | F | F | H | OH | 128-130 |
| 186 | F | F | H | $O\overset{O}{\overset{\|}{C}}NHC_2H_5$ | 99-101 |
| 187 | F | F | H | $OCH_3$ | 75-77 |
| 188 | H | Br | H | COOH | 206-208 |
| 189 | Cl | Cl | H | $COOCH_2CH=CH-C_2F_5$ | 59-61 |
| 190 | H | Br | H | $COOCH_2C\equiv CH$ | 108-110 |
| 191 | H | Br | H | $COO\underset{CH_3}{\underset{\|}{C}}H-COOC_2H_5$ | Wachs |
| 192 | H | Br | H | $CONHCH_2CH_2OCH_3$ | 197-199 |
| 193 | H | Br | H | $COO\underset{CCl_3}{\underset{\|}{C}}H-NH\overset{O}{\overset{\|}{C}}CH(CH_3)_2$ | 196-198 |
| 194 | H | Br | H | $CONH-\underset{CH_3}{\underset{\|}{C}}H-COOC_2H_5$ | 111-113 |

Fortsetzung Tabelle 1

| Bsp. Nr. | X | Y | $R^1$ | Z | Schmp. [°C] |
|---|---|---|---|---|---|
| 195 | H | Br | H | $CH_2OCH_2OCH_3$ | 66-68 |
| 196 | H | Br | H | $CH_2OC(=O)NHCH_3$ | Wachs |
| 197 | H | Br | H | $CH_2OC(=O)CH_3$ | Harz |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

**1. Unkrautwirkung im Vorauflauf**

Samen bzw. Rhizomstücke von mono-und dikotylen Unkrautpflanzen wurden in Plastiktöpfen (Durchmesser: 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der abdeeckerde appliziert.

Nach der Behandlung wurden die Töpfe im Gewächshaus aufgestellt und unter guten Wachstumsbedingungen für die Unkräuter gehalten (Temperatur: 23 plus/minus 2 Grad Celsius; relative Luftfeuchte: 60 - 80 °C).

Die optische Bonitur der Pflanzen-bzw. der Auflaufschäden erfolgte nach dem Auflaufen der Versuchspflanzen nach einer Versuchszeit von 3 - 4 Wochen im Vergleich zu unbehandelten Kontrollen. Wie die Boniturwerte in Tabelle 1 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono-und dikotylen Unkräutern wurden in Plastiktöpfen (Durchmesser = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im

Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 - 800 l/ha auf die grünen Pflanzentiele gesprüht und nach ca. 3 - 4 Wochen Standzeit der Versuchspflanzen im Gewächshaus unter optimalen Wachstumsbedingungen (Temperatur: 23 plus/minus 2 Grad Celsisus, relative Luftfeuchte 60 - 80 %) die Wirkung der Präparate optisch im Vergleich zu unbehandelten Kontrollen bonitiert.

Die erfindungsgemäßen Mittel weisen auch im Nachauflauf eine gute herbizide Wirksamkeit gegne ein breites Spektrum wirkschaftlich wichtiger Ungräser und Unkräuter auf (Tabelle 2).

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Eine Teil der Töpfe wurde sofort wie unter 1 beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweiblättrige Kulturen wie z.B. Soja, Baumwolle, Raps, Zuckerrüben und Kartoffeln im Vor-und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

**Tabelle 2: Vorauflaufwirkung der erfindungsgemäßen Verbindungen**

| Bsp. Nr. | Dosis kg a.i./ha | herbizide Wirkung SIA | CRS | STM | ECG |
|---|---|---|---|---|---|
| 6 | 2,5 | 5 | 5 | 2 | 5 |
| 16 | 2,5 | 4 | 5 | 2 | 5 |
| 27 | 2,5 | 3 | 5 | 5 | 5 |
| 1 | 2,5 | 3 | 5 | 5 | 5 |
| 35 | 2,5 | 5 | 5 | 5 | 4 |
| 2 | 2,5 | 5 | 5 | 5 | 5 |
| 37 | 2,5 | 4 | 5 | 5 | 5 |
| 38 | 2,5 | 2 | 5 | 2 | 3 |
| 41 | 2,5 | 4 | 5 | 5 | 5 |
| 42 | 2,5 | 5 | 5 | 5 | 5 |
| 65 | 2,5 | 5 | 5 | 5 | 5 |
| 66 | 2,5 | 5 | 5 | 5 | 5 |

Fortsetzung Tabelle 2

| Bsp. Nr. | Dosis kg a.i./ha | herbizide Wirkung SIA | CRS | STM | ECG |
|---|---|---|---|---|---|
| 67 | 2,5 | 3 | 4 | 3 | 3 |
| 68 | 2,5 | 5 | 5 | 5 | 5 |
| 69 | 2,5 | 5 | 5 | 2 | 4 |
| 70 | 2,5 | 2 | 5 | 2 | 2 |
| 74 | 2,5 | 5 | 5 | 5 | 5 |
| 75 | 2,5 | 3 | 5 | 3 | 5 |
| 76 | 2,5 | 4 | 5 | 2 | 5 |
| 78 | 2,5 | 5 | 5 | 2 | 5 |
| 79 | 2,5 | 5 | 5 | 2 | 4 |
| 80 | 2,5 | 5 | 5 | 2 | 5 |
| 81 | 2,5 | 5 | 5 | 5 | 5 |
| 82 | 2,5 | 5 | 5 | 5 | 5 |
| 83 | 2,5 | 5 | 5 | 5 | 5 |
| 84 | 2,5 | 5 | 5 | 5 | 5 |
| 85 | 2,5 | 5 | 5 | 2 | 3 |
| 87 | 2,5 | 5 | 5 | 5 | 4 |
| 88 | 2,5 | 5 | 5 | 4 | 5 |
| 86 | 2,5 | 3 | 5 | 2 | 3 |
| 72 | 2,5 | 4 | 5 | 4 | 4 |
| 89 | 2,5 | 5 | 5 | 5 | 5 |
| 90 | 2,5 | 5 | 5 | 5 | 5 |
| 181 | 2,5 | 4 | 5 | 5 | 5 |
| 93 | 2,5 | 5 | 5 | 5 | 5 |
| 91 | 2,5 | 3 | 4 | 4 | 3 |
| 182 | 2,5 | 3 | 4 | 4 | 3 |
| 183 | 2,5 | 5 | 5 | 5 | 4 |
| 184 | 2,5 | 2 | 5 | 5 | 4 |
| 185 | 2,5 | 4 | 5 | 2 | 3 |
| 130 | 2,5 | 5 | 5 | 5 | 5 |
| 186 | 2,5 | 5 | 5 | 4 | 5 |
| 187 | 2,5 | 3 | 5 | 2 | 4 |
| 129 | 2,5 | 4 | 5 | 2 | 3 |
| 196 | 2,5 | 2 | 5 | 3 | 2 |

**Abkürzungen:**
SIA = Sinapis alba, CRS = Chrysanthemum segetum, STM = Stellaria media, ECG = Echinochloa crus-galli, a. i = Aktivsubstanz

**Tabelle 3: Nachauflaufwirkung der erfindungsgemäßen Verbindungen**

| Bsp. | Dosis | herbizide Wirkung | | | |
|---|---|---|---|---|---|
| Nr. | kg a.i./ha | SIA | CRS | STM | ECG |
| 6 | 2,5 | 5 | 3 | 4 | 2 |
| 16 | 2,5 | 5 | 5 | 2 | 4 |
| 27 | 2,5 | 4 | 4 | 4 | 3 |
| 1 | 2,5 | 4 | 5 | 5 | 4 |
| 35 | 2,5 | 5 | 5 | 5 | 5 |
| 2 | 2,5 | 5 | 5 | 5 | 5 |
| 37 | 2,5 | 5 | 4 | 5 | 3 |
| 38 | 2,5 | 5 | 5 | 5 | 3 |
| 41 | 2,5 | 4 | 5 | 4 | 3 |
| 42 | 2,5 | 5 | 5 | 5 | 4 |
| 65 | 2,5 | 5 | 5 | 4 | 4 |
| 66 | 2,5 | 5 | 5 | 4 | 4 |
| 67 | 2,5 | 5 | 5 | 3 | 3 |
| 68 | 2,5 | 5 | 5 | 5 | 5 |
| 69 | 2,5 | 5 | 5 | 4 | 2 |
| 70 | 2,5 | 5 | 5 | 3 | 4 |
| 74 | 2,5 | 5 | 5 | 5 | 5 |
| 75 | 2,5 | 5 | 5 | 3 | 3 |
| 76 | 2,5 | 5 | 5 | 4 | 5 |
| 78 | 2,5 | 5 | 5 | 4 | 5 |
| 79 | 2,5 | 5 | 5 | 4 | 4 |
| 80 | 2,5 | 5 | 5 | 3 | 4 |
| 81 | 2,5 | 5 | 5 | 5 | 5 |
| 82 | 2,5 | 5 | 5 | 5 | 5 |
| 83 | 2,5 | 5 | 5 | 5 | 5 |
| 84 | 2,5 | 5 | 5 | 5 | 5 |
| 85 | 2,5 | 5 | 4 | 3 | 4 |

| Bsp. Nr. | Dosis kg a.i./ha | herbizide Wirkung SIA | CRS | STM | ECG |
|---|---|---|---|---|---|
| 87 | 2,5 | 5 | 5 | 5 | 4 |
| 88 | 2,5 | 5 | 5 | 5 | 5 |
| 86 | 2,5 | 5 | 5 | 4 | 4 |
| 72 | 2,5 | 5 | 5 | 5 | 5 |
| 89 | 2,5 | 5 | 5 | 5 | 5 |
| 90 | 2,5 | 5 | 5 | 5 | 5 |
| 181 | 2,5 | 5 | 5 | 5 | 4 |
| 93 | 2,5 | 5 | 5 | 5 | 5 |
| 91 | 2,5 | 5 | 5 | 5 | 3 |
| 182 | 2,5 | 5 | 5 | 5 | 3 |
| 183 | 2,5 | 5 | 5 | 5 | 5 |
| 184 | 2,5 | 5 | 5 | 5 | 3 |
| 185 | 2,5 | 5 | 4 | 4 | 4 |
| 130 | 2,5 | 5 | 5 | 5 | 5 |
| 186 | 2,5 | 5 | 5 | 4 | 5 |
| 187 | 2,5 | 4 | 3 | 2 | 2 |
| 188 | 2,5 | 4 | 4 | 4 | 2 |
| 129 | 2,5 | 5 | 5 | 4 | 5 |
| 195 | 2,5 | 5 | 5 | 4 | 3 |
| 189 | 2,5 | 4 | 5 | 2 | 2 |
| 190 | 2,5 | 5 | 5 | 3 | 4 |
| 191 | 2,5 | 5 | 5 | 4 | 4 |
| 196 | 2,5 | 5 | 5 | 3 | 4 |
| 197 | 2,5 | 5 | 5 | 4 | 4 |
| 192 | 2,5 | 3 | 3 | 2 | 3 |

Abkürzungen wie Tabelle 2.

## Ansprüche

1. Verbindungen der Formel I oder deren Salze.

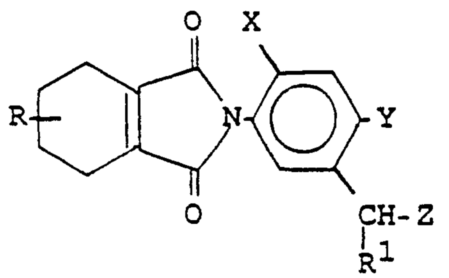

(I)

worin
X = Wasserstoff oder Halogen,
Y = Halogen

Z = 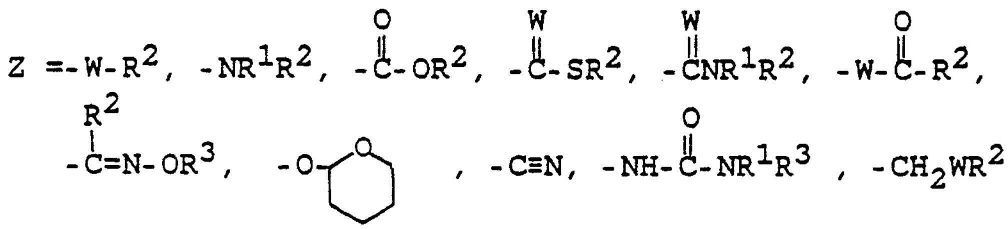 oder

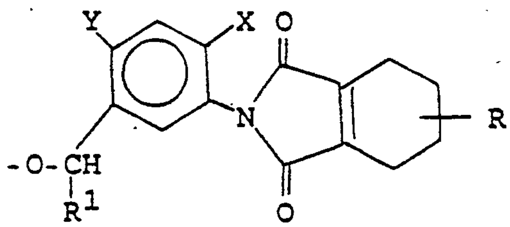

R = H oder $(C_1$-$C_4)$-Alkyl,
R' = Wasserstoff, Hydroxy, $(C_1$-$C_4)$-Alkyl, $(C_1$-$C_4)$-Alkoxy, $(C_1$-$C_4)$-Alkylthio, Amino, $(C_1$-$C_4)$-Alkylamino, $(C_1$-$C_4)$-Dialkylamino oder Cyano,
oder R' und Z zusammen einen Rest -W-$CH_2$-$CH_2$-W-,
$R^2$ = Wasserstoff, $(C_1$-$C_4)$-Alkyl, $(C_2$-$C_4)$-Alkenyl, $(C_2$-$C_4)$-Alkinyl, wobei der Alkyl-, Alkenyl-oder Alkinyl-Rest jeweils durch $(C_3$-$C_7)$-Cycloalkyl, Halogen, $(C_1$-$C_4)$-Alkoxy, $(C_1$-$C_4)$-Alkylthio, Di-$(C_1$-$C_4$-alkyl)amino, Cyano, Nitro, Phenyl, Tri-$(C_1$-$C_4$-alkyl)silyl, Benzyloxy oder durch einen vier-bis siebengliedrigen gesättigten oder ungesättigten ein bis drei Stickstoff-, Sauerstoff-odere Schwefelatome enthaltenden Heterocyclus substituiert sein kann;
$(C_3$-$C_7)$-Cycloalkyl; unsubstituiertes oder durch Halogen, Cyano, $(C_1$-$C_4)$Alkyl, $(C_1$-$C_4)$-Alkoxy oder durch Phenoxy, das durch $CF_3$, Halogen oder $(C_1$-$C_4)$-Alkyl substituiert sein kann, substituiertes Phenyl; einen Rest der Formeln

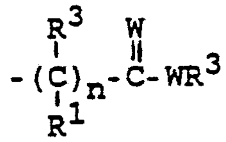, 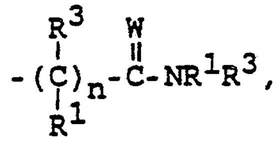, 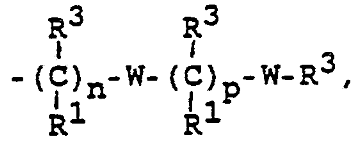,

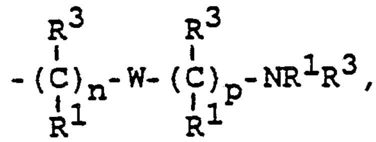, 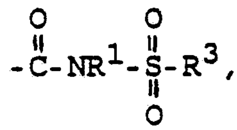, 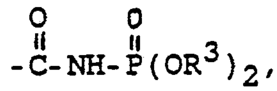,

$-S(O)_pOR^3$ oder 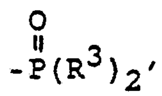,

$R^3$ = Wasserstoff, ($C_1$-$C_4$)-Alkyl, ($C_3$-$C_7$)-Cycloalkyl, ($C_2$-$C_4$)-Alkenyl, ($C_2$-$C_4$)-Alkinyl oder Phenyl, das durch Halogen, Cyano, ($C_1$-$C_4$)-Alkyl oder ($C_1$-$C_4$)-Alkoxy substituiert sein kann,
n = 0, 1 oder 2
p = 1 oder 2 und
W = Sauerstoff oder Schwefel
bedeuten.

2. Verbindungen der Formel I von Anspruch 1 oder deren Salze, worin
X = H, F oder Cl
Y = F, Cl oder Br

Z = $-WR^2$, 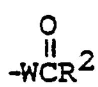 oder 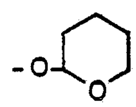, insbesondere $WR^2$,

$R^1$ = H, ($C_1$-$C_4$)-Alkyl oder ($C_1$-$C_4$)-Alkoxy
$R^2$ = H, ($C_1$-$C_4$)-Alkyl, ($C_2$-$C_4$)-Alkenyl, ($C_2$-$C_4$)-Alkinyl, wobei diese Reste durch ($C_1$-$C_4$)-Alkoxy, ($C_1$-$C_4$)-Alkylthio oder Halogen substituiert sein können oder einen Rest der Formeln

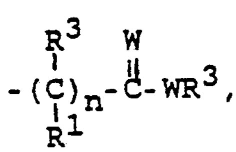, 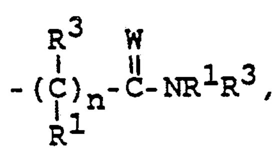, 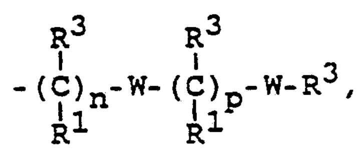,

$R^3$ = H, ($C_1$-$C_4$)-Alkyl oder Phenyl
n = 0, 1 oder 2
p = 1 oder 2 und
W = O oder S bedeuten.

3. Verfahren zur Herstellung der Verbindungen von Formel I gemäß Ansprüchen 1 oder 2, dadurch gekennzeichnet, daß man

a) eine Verbindung der Formel II mit einer Verbindung der Formel III

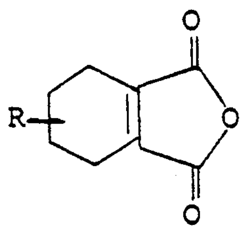

II

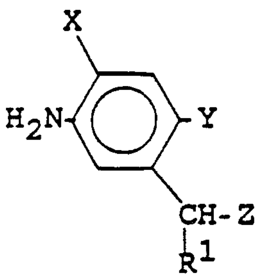

III

oder

b) ein Isocyanat der Formel (IV) mit einer Verbindung der Formel II

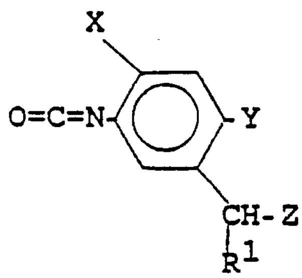

IV

oder

c) eine Verbindung der Formel (V),

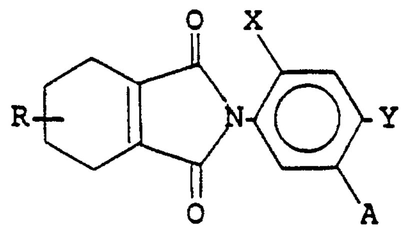

V

worin A = einen Rest der Formeln -CHO, $-CHR^1$-Hal (Hal = Cl oder Br), $-CHR^1-OH$ oder $CHR^1-COOR^3$ bedeutet in an sich bekannter Weise zu den Verbindungen der Formel I derivatisiert uns die unter a) bis c) erhaltenen Verbindungen gegebenenfalls in ihre Salze überführt.

4. Herbizide Mittel, dadurch gekennzeichnet, daß sie eine wirksame Menge einer Verbindung der Formel I von Ansprüchen 1 oder 2 oder deren Salze enthalten.

5. Verwendung von Verbindungen der Formel I von Ansprüchen 1 oder 2 oder deren Salze als Herbizide.

6. Verfahren zur Bekämpfung von unerwünschten Pflanzen, dadurch gekennzeichnet, daß man auf diese oder die Nutzflächen eine wirksame Menge einer Verbindung der Formel I von Ansprüchen 1 oder 2 oder deren Salze appliziert.

Patenansprüche für folgenden Vertragsstaat: ES:

1. Verfahren zur Herstellung der Verbindungen der Formel I oder deren Salze.

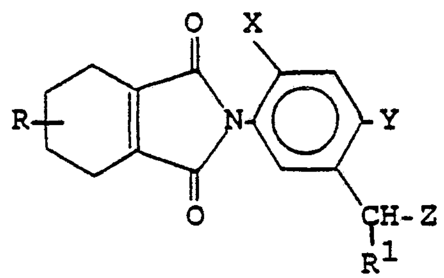

(I)

worin
X = Wasserstoff oder Halogen,
Y = Halogen

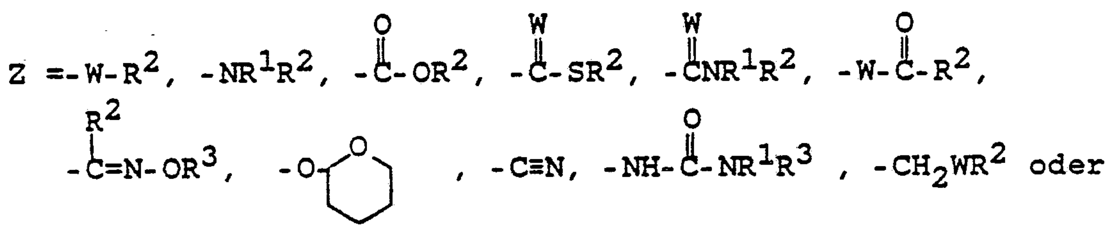

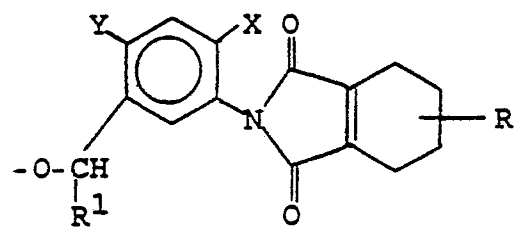

R = H oder $(C_1-C_4)$-Alkyl
$R^1$ = Wasserstoff, Hydroxy, $(C_1-C_4)$-Alkyl, $(C_1-C_4)$-Alkoxy, $(C_1-C_4)$-Alkylthio, Amino, $(C_1-C_4)$-Alyklamino, $(C_1-C_4)$-Dialkylamino oder Cyano,
oder $R^1$ und Z zusammen einen Rest $-W-CH_2-CH_2-W-$,
$R^2$ = Wasserstoff, $(C_1-C_4)$-Alkyl, $(C_2-C_4)$-Alkenyl, $(C_2-C_4)$-Alkinyl, wobei der Alkyl-, Alkenyl-oder Alkinyl-Rest jeweils durch $(C_3-C_7)$-Cycloalkyl, Halogen, $(C_1-C_4)$-Alkoxy, $(C_1-C_4)$-Alkylthio, Di-$(C_1-C_4$-alkyl)amino, Cyano, Nitro, Phenyl, Tri-$(C_1-C_4$-alkyl)silyl, Benzyloxy oder durch einen vier-bis siebengliedrigen gesättigten oder ungesättigten ein bis drei Stickstoff-, Sauerstoff-odere Schwefelatome enthaltenden Heterocyclus substituiert sein kann;
$(C_3-C_7)$-Cylcoalkyl; unsubstituiertes oder durch Halogen, Cyano, $(C_1-C_4)$Alkyl, $(C_1-C_4)$-Alkoxy oder durch Phenoxy, das durch $CF_3$, Halogen oder $(C_1-C_4)$-Alkyl substituiert sein kann, substituiertes Phenyl; einen Rest der Formeln

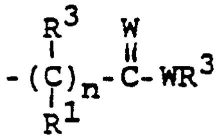, 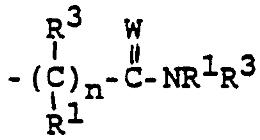, 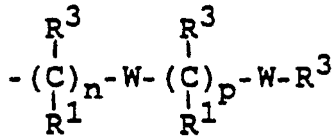,

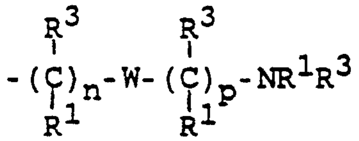, 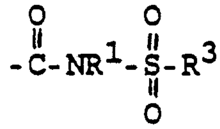, 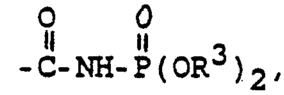,

$-S(O)_pOR^3$ oder 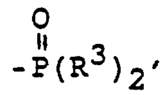,

$R^3$ = Wasserstoff, $(C_1-C_4)$-Alkyl, $(C_3-C_7)$-Cycloalkyl, $(C_2-C_4)$-Alkenyl, $(C_2-C_4)$-Alkinyl oder Phenyl, das durch Halogen, Cyano, $(C_1-C_4)$-Alkyl oder $(C_1-C_4)$-Alkoxy substituiert sein kann,

n = 0, 1 oder 2

p = 1 oder 2 und

W = Sauerstoff oder Schwefel

bedeuten, dadurch gekennzeichnet, daß man

a) eine Verbindung der Formel (II) mit einer Verbindung der Formel (III)

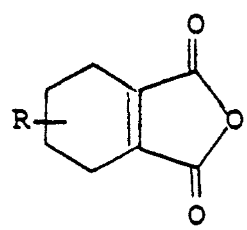 II 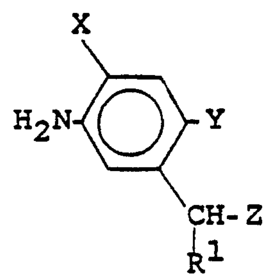 III

oder

b) eine Isocyanat der Formel (IV) mit einer Verbindung der Formel II

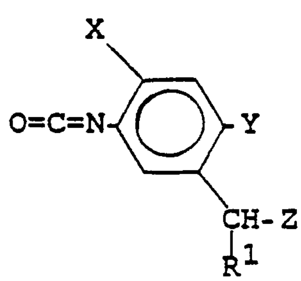 IV

oder

c) eine Verbindung der Formel (V),

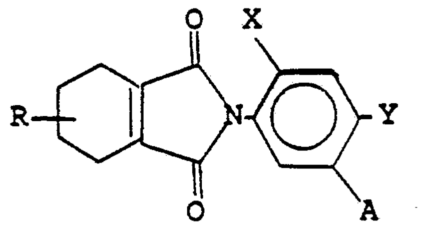

V

worin A = einen Rest der Formeln -CHO, $-CHR^1$-Hal (Hal = Cl oder Br), $-CHR^1-OH$ oder $CHR^1-COOR^3$ bedeutet in an sich bekannter Weise zu den Verbindungen der Formel I derivatisiert uns die unter a) bis c) erhaltenen Verbindungen gegebenenfalls in ihre Salze überführt.

2. Verfahren gemäß Anspruch 1, dadurch gekennzeichnet, daß in Formel I von Anspruch 1

X = H, F oder Cl

Y = F, Cl oder Br

Z = $-WR^2$, 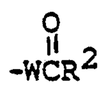 oder 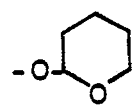, insbesondere $WR^2$,

$R^1$ = H, $(C_1-C_4)$-Alkyl oder $(C_1-C_4)$-Alkoxy

$R^2$ = H, $(C_1-C_4)$-Alkyl, $(C_2-C_4)$-Alkenyl, $(C_2-C_4)$-Alkinyl, wobei diese Reste durch $(C_1-C_4)$-Alkoxy, $(C_1-C_4)$-Alkylthio oder Halogen substituiert sein können, oder einen Rest der Formeln

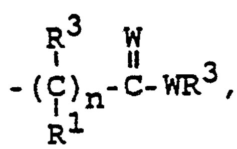, 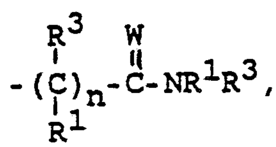, 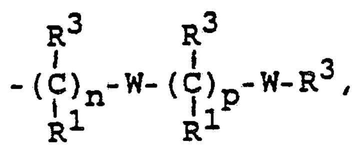,

$R^3$ = H, $(C_1-C_4)$-Alkyl oder Phenyl

n = 0, 1 oder 2

p = 1 oder 2 und

W = O oder S bedeuten.

3. Verfahren zur Bekämpfung von unerwünschten Pflanzen, dadurch gekennzeichnet, daß man auf diese oder die Nutzflächen eine wirksame Menge einer Verbindung der Formel I von Ansprüchen 1 oder 2 oder deren Salze appliziert.